Europäisches Patentamt

European Patent Office

Office européen des brevets

(11) Publication number: **0 377 051**
**A1**

# EUROPEAN PATENT APPLICATION
## published in accordance with Art. 158(3) EPC

(21) Application number: 89906474.5

(22) Date of filing: 06.06.89

(86) International application number:
**PCT/JP89/00569**

(87) International publication number:
**WO 89/11830 (14.12.89 89/29)**

(51) Int. Cl.⁵: **A61B 17/36**

(30) Priority: 06.06.88 JP 138758/88

(43) Date of publication of application:
**11.07.90 Bulletin 90/28**

(84) Designated Contracting States:
**DE FR GB IT NL**

(71) Applicant: **SUMITOMO ELECTRIC INDUSTRIES, LTD**
**5-33, Kitahama 4-chome Chuo-ku**
**Osaka 541(JP)**

(72) Inventor: **SOGAWA, Ichiro Osaka Works of Sumitomo Electr.**
**Ind., Ltd. 1-3, Shimaya 1-chome Konohana-ku Osaka-shi Osaka 554(JP)**
Inventor: **NIWA, Shin-ichiro Osaka Works of Sumitomo Electr.**
**Ind., Ltd. 1-3, Shimaya 1-chome Konohana-ku Osaka-shi Osaka 554(JP)**
Inventor: **YOTSUYA, Koro Osaka Works of Sumitomo Electr.**
**Ind., Ltd. 1-3, Shimaya 1-chome Konohana-ku Osaka-shi Osaka 554(JP)**
Inventor: **UEMIYA, Takafumi Osaka Works of Sumitomo Electr.**
**Ind., Ltd. 1-3, Shimaya 1-chome Konohana-ku Osaka-shi Osaka 554(JP)**
Inventor: **KANAZAWA, Shin-ichi Osaka Works of Sumitomo Electr**
**Ind., Ltd. 1-3, Shimaya 1-chome Konohana-ku Osaka-shi Osaka 554(JP)**

(74) Representative: **Lehn, Werner, Dipl.-Ing. et al**
**Hoffmann, Eitle & Partner Patentanwälte**
**Arabellastrasse 4**
**D-8000 München 81(DE)**

(54) **LASER-AIDED INTRAVASCULAR OPERATION EQUIPMENT.**

(57) Blood vessel operation equipment using laser. A catheter (2) is equipped with an endoscopic fiber (21) for endoscopically viewing the diseased part in the blood vessel of a living body, a fiber (24) for

irradiating the diseased part with a laser beam, a transparent drug conducting path for removing blood, and a balloon (6) provided at the end portion. The laser device (1) is suitably controlled based upon the data from an image analyzer (3) and a laser beam is emitted, making it possible to carry out the therapy while confirming the diagnostic condition at all times without needing labor such as exchanging the fibers. Further, output signals of an electrocardiograph (71) are monitored to prevent in advance the stanching or adverse effect upon the living body caused by irradiation with the laser beam.

*Fig. 1*

SPECIFICATION

LASER OPERATING DEVICE FOR INTRAVASCULAR SURGERY

FIELD OF THE INVENTION

The present invention relates to a laser operating device for intravascular surgery, and more in particular to a laser operating device for intravascular surgery which is used for removing a diseased part in a blood vessel using laser light.

BACKGROUND OF THE INVENTION

In the prior art, there have been contrived various kinds of medical treatment diagnosis devices and methods thereof for removing e.g. a stenosis or occlusion of a blood vessel and an atheroma due to such as an arteriosclerosis.

A bypass operation is the most assured treatment method in which a lesion is completely removed by replacing a blood vessel which includes a diseased part with one of patient's own blood vessels or with an artificial blood vessel for example. However, since this method is followed by a surgery in which tissue is cut open, the vital body is subjected to a burden and a large amount of costs is needed for the treatment. Moreover, although a drug treatment is also adopted, it is effective only for solution of a thrombus and it has been difficult to remove an arteriosclerosis focus.

Therefore, there has been recently adopted a treatment that a catheter is inserted into a blood vessel from the outside of a body and reached a diseased part so as to directly remove a cause of an obstacle.

One is a treatment that a balloon catheter having a balloon attached on its distal tip portion is used and the balloon is expanded when it is reached the diseased part so that the stenosis of the blood vessel is mechanically expanded. However, since the stenosis is simply expanded, e.g. the diseased focus of the arteriosclerosis or thrombus which causes a stenosis can not be removed and a probability of a relapse of a disease in a short period is high. Moreover, in the cases that the blood vessel is entirely occluded and that the arteriosclerosis is so advanced as to cause a calcification, it becomes difficult to treat with a balloon.

The other is a method using laser light such as YAG laser or argon laser, wherein a metallic or ceramic chip attached to the tip of the catheter is heated by the laser light radiated from the tip of an optical fiber so that the heated chip is pressed onto the diseased part so as to burn out the diseased part. According to this method, though the diseased part can be removed, the control of the light heating power is difficult and if the chip is overheated, a normal vessel wall is damaged or carbonized so that there may cause a new risk of vascular perforation or a new re- stenosis. Moreover, in case the vessel is tortuous or completely occluded, it is not available because the chip can not be inserted.

Therefore, it is also adopted that the laser light from such as YAG laser, argon laser and excimer laser is directly projected to the diseased part from the tip of the fiber so as to vaporized the diseased part. Since the portion having the laser light projected thereon is directly vaporized, the laser light is available also for a completely occluded diseased part and upon controlling the output of the laser light source, it is possible to treat with high accuracy.

By the way, in the case of diagnosis and treatment of a diseased part in a vessel by means of the intravascular laser operating device of a direct radiation type mentioned above, first, endoscopic optical fiber and illumination optical fiber are inserted and a balloon for fixing the tip of the catheter and stopping the blood flow attached to the tip portion of the catheter is expanded so as to stop the blood flow and liquid (blood removing transparent drug liquid) having little loss in the range of the wave length of the laser light in use is charged and replaced with blood so that the condition in the blood vessel is observed under the condition that the vision is made transparent. Subsequently, after the diagnosis of the diseased part is performed, the fibers are replaced with a laser light applying fiber and in this condition, the laser light is applied to remove the diseased part.

In this case, since it is desirable that the treatment time is as short as possible considering the safety for the tissue, the above mentioned endoscopy, replace of the fiber and laser projection must be performed quickly and if it takes much time, the occlusion by the balloon and charge of the blood removal transparent drug liquid must be released so that the operation must be done over again. If the treatment is performed for a long time unconscious of the time lapse, there may occur a menace to the safety of the tissue.

Moreover, while the laser is being projected, since it is impossible to observe the diseased part at the same time, it is confirmed after the operation whether or not the projection is precisely performed with proper power, and in case the projection is not proper and precise, the processes such as the endoscopy and projection are repeated over and over again, resulting in the patient is subjected to burden.

Accordingly, in the above mentioned intravascular laser operating device of a direct applying type, it has been difficult to secure the quickness and sureness of the treatment and the safety of the tissue.

Moreover, there have been problems that, when performing an endoscopy of the inside of a blood vessel, since the endoscopic image is trembled because of the trembling of the tip of the catheter following to the heart beat, the diagnosis becomes difficult, and furthermore that the position to be applied by the laser light is indefinite.

The present invention has been made considering the problems mentioned above and has its object to provide an intravascular laser operating device capable of treatment securing the safety for tissue with rapidity and soundness and capable of obtaining a stable endoscopic image.

DISCLOSURE OF THE INVENTION

In order to accomplish the object mentioned above, the intravascular laser operating device of the present invention comprises an endoscopic fiber for endoscopy of a diseased part in a vital blood vessel and a laser light applying fiber for applying laser light to the diseased part, and further comprises; a catheter having a blood removal transparent drug liquid passage path and a balloon attached to the tip portion, a laser device for applying laser light to the laser light applying fiber, an image diagnosis device for obtaining the image data representing the condition of the inside of a blood vessel based on the output light from the endoscopic fiber, a monitor unit for processing the output signal from an electrocardiograph and monitoring the condition of the patient, and control means for controlling at least one or more of the constriction, dilation of the balloon and charge of the blood removal transparent drug liquid and laser projection based on the monitor output of the monitor unit.

According to the intravascular laser operating device constituted as described above, under the condition that the catheter is inserted into the blood vessel to remove the blood due to the occlusion by the balloon and/or charge of the drug liquid, the condition of the diseased part in the blood vessel can be seen by means of the image diagnosis device. And according to the diagnosis result based on the endoscopy, the projection condition such as the output and pulse width of the laser light is set, whereby the treatment is performed with laser light. In this case, since there is not required a time for replacing the fibers, the laser device is driven as it is after the endoscopy, thereby advancing to the treatment with laser light. In this case, the endoscopy as well as the laser treatment can be continued at the same time.

If the patient is badly affected due to such as a delayed treatment, there appears an abnormal change thereof in the output signal of the electrocardiograph first. And the abnormal change can be detected by the monitor device and on the basis of the monitor output of the monitor device, the constriction of the balloon or the charge of the transparent drug liquid can be stopped. Therefore, the blood current is started again and the bad influence onto the patient can be previously avoided.

Moreover, if the laser light is projected to the intravascular wall, there occurs such as a spasm of the blood vessel and the condition of the blood current becomes worse, but also in this case, since there appears an abnormal change in the output signal of the electrocardiograph, the projection of the laser light can be stopped on the basis of the monitor output of the monitor device also in such a case.

Furthermore, when performing an endoscopy of the inside of the blood vessel, the image is obtained synchronously with the heart beat detected by the electrocardiogram and displayed on the display unit, whereby an endoscopic image with a constant view can be obtained. Moreover, also when the laser is projected, the laser is projected synchronously with the heart beat detected by the electrocardiogram, whereby the laser can be projected to a constant position.

As described above, the laser treatment can be quickly performed securing the safety of the patient.

BRIEF DESCRIPTION OF THE DRAWINGS

Fig. 1 is a schematic diagram showing an embodiment of an intravascular laser operating device,

Fig. 2 is a sectional view of a catheter,

Fig. 3 is a block diagram showing a laser device, drive control unit, image device and monitor unit of the intravascular laser operating device, and

Fig. 4 is a flow chart showing an operating process of the intravascular laser operating device.

OPTIMUM EMBODIMENT OF THE INVENTION

An embodiment of the present invention is explained hereinafter with reference to the drawings.

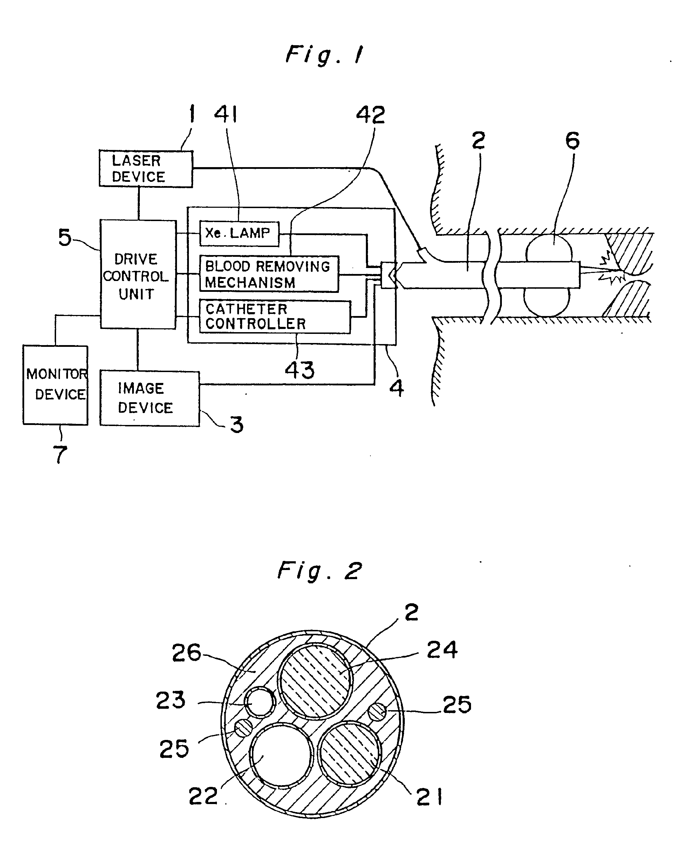

As shown in Fig. 1, the intravascular laser operating device of the present embodiment comprises a laser device (1), an operating catheter (2) having an endoscopic fiber for endoscopy of a diseased part, an illumination light guide, a blood removal transparent drug liquid passage hole, a balloon dilation fluid passage hole, laser light illumination fiber and tip control wire, an image device (3) for displaying a form of an endoscopic image and displaying a fluorescent spectrum analysis and recording the image data, and a drive control unit (5) for applying an illumination light to said catheter (2) through an interface portion (4) and supplying such as liquid. Moreover, (6) is a fixing balloon for occluding the blood vessel provided on the tip of the catheter (2), and (7) denotes a monitor device monitoring an abnormal change of an electrocardio voltage signal generated at an electrode attached to a predetermined part of a patient.

Fig. 2 is a sectional view of the catheter (2) which is made in the manner that, endoscopic fibers (21) and laser light illumination fibers (24) and a tip control wire (25) are bundled to be fixed in transparent medium (26), forming a liquid removing transparent drug liquid passage hole (22), a balloon dilation fluid passage hole (23), having the surface coated with thin layer. Moreover, said transparent medium (26) serves as the illumination light guide.

Moreover, it is not always necessary that said endoscopic fibers (21) and laser light projecting fibers (24) are fixed in the transparent medium (26), but there may be provided a proper inserting hole in the transparent medium (26), thereby removably inserting the fibers (21) and (24) therethrough.

The endoscopic fiber (21) is made of materials having little dispersion, accomplishing the high accuracy of the both edge optical systems particularly in order to accomplish the high quality of the image forming. The laser light projecting fiber (24) is made of materials such as quartz with good transmittance through which ultraviolet rays can be transmitted with high energy density and with low loss and the end surface thereof is processed with high accuracy in order to suppress the heat generation at the end surface. The transparent medium (26) as the illumination light guide is made of visible light transmittable materials with good flexibility such as multi-components group glass, plastic resin and rubber and the illumination light is projected from the tip section of the catheter (2). Using the tip control wire (25) which is controlled by a catheter controller (43), the tip of the catheter (2) is guided to a diseased part to be described later in a manner of opposing to the diseased part.

The outer diameter of the catheter (2) accommodating the respective components (21) to (26) mentioned above is made extremely thinned diameter of a few milli meters, preferably less than 1.5 mm. Therefore, it becomes possible to easily reach any part in the blood vessel by the guide of the catheter controller (43).

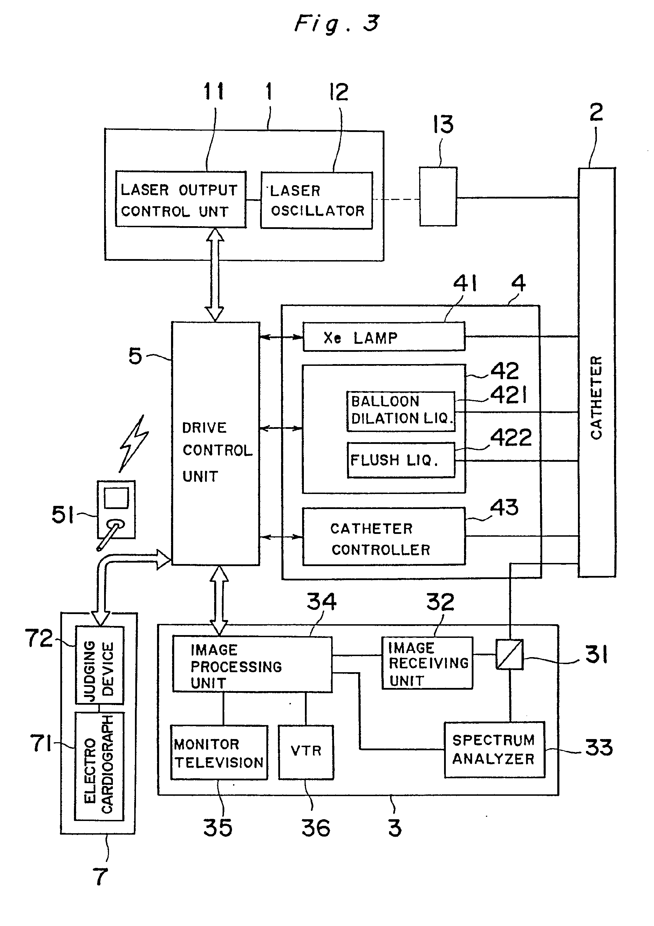

Fig. 3 is a diagram showing the details of the laser device (1), interface portion (4), drive controller (5), image device (3) and monitor device (7) mentioned above.

The laser device comprises of a laser output controller (11) and laser oscillating unit (12), wherein the laser output controller (11) controls the power and pulse intervals of the laser light projected from the laser oscillating unit (12). The laser oscillating unit (12) is composed of a pulse oscillation excimer laser of noble gas halide such as XeCl, KrF and ArF. Moreover, (13) denotes a connecting portion for connecting the projected laser light to the light leading fiber (24), which is composed of a minute optical system having little loss.

The interface portion (4) comprises a Xe lamp (41) for applying visible light to said illumination light guide (26), a blood removing mechanism (42) for charging balloon dilation liquid (421) (such as isotonic sodium chloride solution) and blood removal transparent drug liquid (422) (liquid having little loss in the range of the wave length of the used laser) into the balloon dilation fluid passage hole (23) and blood removal transparent drug liquid passage hole (22), and a catheter controller (43) having an operation mechanism for operating the control wire (25), which are respectively controlled by the drive control unit (5). That is to say, the drive control unit (5) controls said catheter controller (43) so as to reach the catheter (2) to a desired portion, thereby performing the ON/OFF control of the Xe lamp (41) and the charge control of the respective liquid by the blood removing mechanism (42). Besides this, the drive control unit (5) sends a control signal to the laser output controller (11) so as to control the ON/OFF, power and pulse intervals of the laser output. In addition, the drive control unit (5) itself may be operated by a person monitoring the image device (3) or may be automatically operated by accommodating such as a microcomputer so as to produce a predetermined order signal based on a predetermined signal supplied from the image device (3). When a person performs, it may be controlled by a remote control unit (51) from an operating table for example.

The image device (3) comprises a division optical system (31) dividing an image light generated from the endoscopic fiber (21), image receiving unit (32) receiving one of the divided light by CCD elements, spectrum analyzing unit (33) obtaining the components of the fluorescent spectrum of the other divided light, image processing unit (34) compensating the output signals of the image receiving unit (32) and the spectrum analyzing unit (33), monitor television (35) displaying the processed image signal on the screen of the television, and VTR (36) for recording the image.

The monitor device (7) is composed of electrocardiograph (71) for reading the voltage signal from the electrode attached to a predetermined part of the patient and a judgment device (72) for judging the abnormal change (such as flattening, negative variation and irregularity of period of T wave) of the output signal based on the output signal of the electrocardiograph (71) and for supplying the order signal for controlling the blood removing mechanism (42) and the laser device (1) to the drive control unit (5) when in the abnormal time. In addition, the judgment device (72) may be accommodated in the drive control unit (5) and if the drive control unit (5) comprises a microcomputer, it is further preferable because the judgment control can be performed by using the microcomputer.

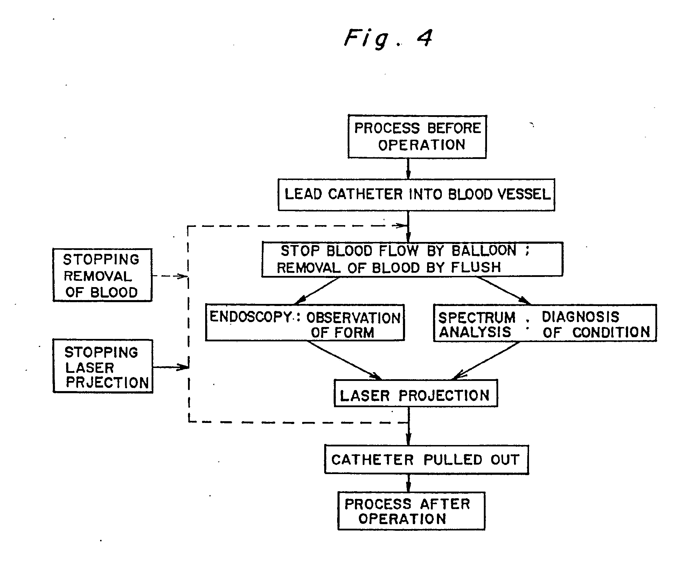

Next, the operating process of said intravascular laser operating device is explained with reference to Fig. 4. First, as the process before operation, after the disinfection of the catheter inserting portion, anesthetization and drug supply are performed, the catheter controller (43) is driven through the drive control unit (5) so that the catheter (2) is guided into a predetermined blood vessel (such as a coronary artery). Subsequently, the balloon dilation liquid (421) is charged into the balloon dilation fluid passage hole (23) so that the balloon is expanded for stopping blood flow and the tip of the catheter (2) is fixed in the blood vessel by the balloon (6). Subsequently, the blood removing transparent drug liquid (422) is quickly charged into the blood removing transparent drug liquid passage hole (22) so that the blood in the lower stream below the occluded portion is replaced to be made transparent. And upon diagnosing the diseased part observing by the monitor television (35), if there is no diseased part, the balloon (6) is constricted to recover the flow of the blood and the catheter (2) is advanced to the other portion. At this time, also the image can be taken by synchronizing with the heart beat detected by the electrocardiogram. If there is a diseased part, the laser device is driven and the laser light is projected so as to destroy the diseased part. At this time, the laser projection can be also performed synchronizing with the heart beat detected by the electrocardiogram. As the laser light, since there is used ultraviolet rays which is largely absorbed by the tissue, using a pulse light with a large peak power, therefore the light does not permeate to the deep of the tissue at the same time and also the range of the heat damage affectable around the projected portion can be limited, therefore, the delicate treatment can be performed with good precision. At this time, since the laser light can be projected using the endoscopic fiber (21) while observing the monitor television (35), it can be immediately judged whether or not the diseased part is completely destroyed. If not completely destroyed, the laser light is projected once more.

Then, if there seems to be a time lapse, the balloon (6) is once constricted to release the occlusion (the release of occlusion is performed because it is undesirable to keep the occlusion for a long time), and subsequently the occlusion is performed once more and the blood is replaced with the blood removing transparent drug liquid (422) and the laser light is projected.

If being unconscious of the time lapse and there occurs an abnormal change in the detection voltage of the electrocardiograph (71), it can be immediately judged by the judgment device (72), therefore, command signals can be generated to the drive control unit (5) for controlling the blood removing mechanism (42) and the laser device (1). Hereby the hemostatic condition is released and/or the projection of the laser light is stopped.

The processes as mentioned above are repeated until the diseased part is completely destroyed. If the diseased part is completely destroyed, the balloon (6) is constricted and the occlusion is released and then the catheter (2) is pulled out. Subsequently, a necessary process after operation is performed and the operation is finished.

In addition, in the process as mentioned above, instead of observing by the monitor television (35), spectrum analyzing unit (33) may be used so that the spectrum diagnosis of the endoscopic light is performed so that the condition of the diseased part may be judged on the basis of the fluorescent spectrum.

Moreover, the processed result in the image device (3) may be judged by such as a microcomputer and all of the operations including the laser projection can be automatically and mechanically performed.

As described above, according to the intravascular laser operating device of the present invention, since both of the endoscopic fiber and laser light projection fiber are comprised in the catheter and the laser light can be projected by properly controlling the drive of the laser device depending on the information from the image diagnosis device, therefore, there is no need to switch the fibers and the treatment can be advanced while confirming the diagnosis condition all the time. Moreover, while monitoring the output signal of the electrocardiograph, the bad influence on the patient due to the occlusion of the blood vessel or the laser projection can be previously prevented. Furthermore, upon taking the endoscopic image synchronizing with the heart beat detected by the electrocardiogram, a stable endoscopic image can be obtained without any trembling. Moreover, upon projecting the laser synchronized with the heart beat detected by the electrocardiogram, the laser can be correctly projected to a predetermined position. Accordingly, there can be obtained a specific effect that the diseased part can be quickly treated with sureness keeping the safety of the patient.

WHAT IS CLAIMED IS:

(1)   An intravascular laser operating device comprising;

a catheter having endoscopic fibers for endoscopy of a diseased part in a blood vessel and laser light projecting fibers for projecting laser light to the diseased part and also having a blood removing transparent drug liquid passage path and a balloon provided on the tip portion thereof,

a laser device applying laser light to said laser light projecting fibers,

an image diagnosis device for obtaining image data representing the condition of the inside of the blood vessel depending on the output light from the endoscopic fibers,

a monitor device for processing the output signal from an electrocardiograph and monitoring the condition of the tissue, and

control means controlling at least one or more of the constriction and dilation of the balloons, charge of the blood removing transparent drug liquid and laser projection.

## Fig. 1

| | |
|---|---|
| 1 | LASER DEVICE |
| 5 | DRIVE CONTROL UNIT |
| 41 | Xe. LAMP |
| 42 | BLOOD REMOVING MECHANISM |
| 43 | CATHETER CONTROLLER |
| 3 | IMAGE DEVICE |
| 7 | MONITOR DEVICE |

## Fig. 2

*Fig. 3*

# Fig. 4

```
                              ┌──────────────────┐
                              │ PROCESS BEFORE   │
                              │   OPERATION      │
                              └────────┬─────────┘
                                       │
                    ┌──────────────────▼──────────────────┐
                    │  LEAD CATHETER INTO BLOOD VESSEL     │
                    └──────────────────┬──────────────────┘
                                       │
                    ┌──────────────────▼──────────────────┐
                    │  STOP BLOOD FLOW BY BALLOON ;        │
                    │  REMOVAL OF BLOOD BY FLUSH           │
                    └──────┬────────────────────────┬──────┘
                           │                        │
        ┌──────────────────▼──────┐    ┌────────────▼──────────────┐
        │ ENDOSCOPY : OBSERVATION │    │ SPECTRUM .  DIAGNOSIS     │
        │            OF FORM      │    │ ANALYSIS ·  OF CONDITION  │
        └──────────────┬──────────┘    └─────────┬─────────────────┘
                       │                          │
                    ┌──▼──────────────────────────▼──┐
                    │      LASER PROJECTION           │
                    └────────────────┬────────────────┘
                                     │
                    ┌────────────────▼────────────────┐
                    │      CATHETER PULLED OUT         │
                    └────────────────┬────────────────┘
                                     │
                    ┌────────────────▼────────────────┐
                    │      PROCESS AFTER              │
                    │      OPERATION                  │
                    └─────────────────────────────────┘
```

┌──────────────┐
│ STOPPING     │
│ REMOVAL      │
│ OF BLOOD     │
└──────────────┘

┌──────────────┐
│ STOPPING     │
│ LASER        │
│ PRJECTION    │
└──────────────┘

# INTERNATIONAL SEARCH REPORT

International Application No  PCT/JP89/00569

## I. CLASSIFICATION OF SUBJECT MATTER (if several classification symbols apply, indicate all) 6

According to International Patent Classification (IPC) or to both National Classification and IPC

Int. Cl⁴    A61B17/36

## II. FIELDS SEARCHED

Minimum Documentation Searched 7

| Classification System | Classification Symbols |
|---|---|
| IPC | A61B17/36, 10/00, A61N5/06 |

Documentation Searched other than Minimum Documentation
to the Extent that such Documents are Included in the Fields Searched 8

| | |
|---|---|
| Jitsuyo Shinan Koho | 1968 – 1989 |
| Kokai Jitsuyo Shinan Koho | 1971 – 1989 |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| X | JP, A, 59-172621 (Sumitomo Electric Industries, Ltd.) 29 September 1984 (29. 09. 84) (Family : none) | 1 |
| Y | JP, A, 62-246359 (Fuji Photo Optical Co., Ltd.) 27 October 1987 (27. 10. 87) (Family : none) | 1 |
| Y | JP, A, 62-277933 (Olympus Optical Co., Ltd.) 2 December 1987 (02. 12. 87) (Family : none) | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing dr   or priority date and not in conflict with the application but cit to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 9, 1989 (09. 08. 89) | August 28, 1989 (28. 08. 89) |
| International Searching Authority | Signature of Authorized Officer |
| Japanese Patent Office | |

Form PCT/ISA/210 (second sheet) (January 1985)